# EUROPÄISCHE PATENTSCHRIFT

(11) **EP 1 646 419 B1**
(45) Veröffentlichungstag und Bekanntmachung des Hinweises auf die Patenterteilung: **10.04.2013**
(21) Anmeldenummer: 04740878.6
(22) Anmeldetag: 09.07.2004
(51) Int. Cl.: A61M 16/06, A61M 16/20

(54) **ATEMMASKENANORDNUNG**
RESPIRATORY MASK ARRANGEMENT
ENSEMBLE D'UN MASQUE RESPIRATOIRE

(30) Priorität: 09.07.2003 DE 10331134; 30.07.2003 DE 10335162
(43) Veröffentlichungstag der Anmeldung: 19.04.2006
(60) Teilanmeldung: 10180729.5
(73) Patentinhaber: ResMed R&D Germany GmbH, 82152 Martinsried (DE)
(72) Erfinder: MADAUS, Stefan, 82152 Krailling (DE); STAUFFENBERG, Caspar, Graf, 82131 Gauting (DE); VÖGELE, Harald, 82131 Gauting (DE)
(74) Vertreter: Vossius & Partner
(86) Internationale Anmeldenummer: PCT/EP2004/007599
(87) Internationale Veröffentlichungsnummer: WO 2005/004963

(56) Entgegenhaltungen:
- EP-A- 0 267 428
- EP-A- 0 697 225
- EP-A- 1 314 445
- WO-A-00/78381
- WO-A-01/32250
- WO-A-02/32491
- US-A- 5 465 712
- US-A1- 2001 032 648

## Beschreibung

### Gebiet der Erfindung

Die Erfindung betrifft eine Atemmaskenanordnung wie sie beispielsweise im Rahmen einer CPAP-Therapie zur Behandlung schlafbezogener Atmungsstörungen Anwendung finden kann. Weiterhin betrifft die Erfindung auch eine Kopfbandanordnung für eine Atemmaske und eine Atemgasableitungseinrichtung für eine Atemmaskenanordnung, zur Ableitung von CO2 befrachtetem Atemgas.

### Hintergrund der Erfindung

Im Rahmen der genannten CPAP-Therapie kann einem Patienten über eine Atemmaskenanordnung ein atembares Gas, insbesondere Umgebungsluft auf einem Druckniveau zugeführt werden, das über dem Umgebungsdruckniveau liegt. Durch das unter Druck stehende Atemgas kann eine pneumatische Schienung der oberen Atemwege erreicht werden und hierdurch etwaigen Obstruktionen vorgebeugt werden. Im Rahmen der Durchführung einer Druckbeatmungs- bzw. CPAP-Therapie werden die zur Zufuhr des Atemgases erforderlichen Atemmaskenanordnungen üblicherweise über die gesamte Schlaf oder Ruhephase des Patienten hinweg von diesem getragen. Die Atemmaskenanordnung stützt sich üblicherweise über eine Dichtlippenzone im Umgebungsbereich der Nase des Maskenanwenders sowie über eine Stimauflageeinrichtung im Stirnbereich des Maskenanwenders ab. Die zur Applikation der Atemmaskenanordnung erforderlichen Haltekräfte können durch eine Fixiereinrichtung, die zum Beispiel ein um den Hinterkopf des Maskenanwenders herumgeführtes Kopfband aufweist, aufgebracht werden. Die Ableitung des ggf. mit CO2 befrachteten Atemgases aus dem Innenbereich der Atemmaske kann über Bohrungen erfolgen, deren Durchgangsquerschnitt derart festgelegt ist, dass ein hinreichend großer Abstrom des Atemgases ermöglicht ist.

Die EP 1 314 445 A1 zeigt eine Atemmaske mit einem Gasabströmventil. In einer Ausführungsform besteht das Ventil aus einer dünnen luftdurchlässigen Membran, wobei die Membran dünner ist als ca. 0.5 mm. Die Membran kann aus wasserabweisendem Material wie Polytetrafluorethylen (PTFE) bestehen. Die Membran kann auch aus expandiertem PTFE hergestellt sein. In einer alternativen Ausführungsform beinhaltet die Ventilanordnung ein Ventil aus rostfreiem Stahl.

Eine Gaszuführleitung lehrt die EP 0 697 225 A2. Die an eine Atemmaske zur künstlichen Beatmung eines Patienten anschließbare Gaszuführleitung weist einen Abschnitt aus einem porösen oder feinporigen Werkstoff auf, der auswechselbar in die Gaszuführung einfügbar ist und über den Überschußluft und die vom Patienten ausgeatmeten Gase an die Umgebung abgegeben werden.

### Aufgabe der Erfindung

Der Erfindung liegt die Aufgabe zugrunde, Lösungen bereitzustellen durch welche eine zuverlässige Ableitung von CO₂ befrachtetem Atemgas aus dem Innenbereich einer Atemmaskenanordnung in vorteilhafter Weise gewährleistet ist.

### Erfindungsgemäße Lösung

Die Aufgabe der vorliegenden Anmeldung wird gelöst durch den Gegenstand der geltenden Ansprüche.

Gemäß einem ersten Aspekt wird diese Aufgabe erfindungsgemäß gelöst, durch eine Atemmaskenanordnung mit einer Dichtlippeneinrichtung zur Auflage auf der Gesichtsfläche eines zur Maskenanwenders, einer Abdeckeinrichtung die im Zusammenspiel mit der Dichtlippeneinrichtung einen Maskeninnenraum definiert, und einer Atemgasleitungseinrichtung zur Zuleitung von Atemgas zu jenem von der Abdeckeinrichtung definierten und mit der Nasen- und/oder Mundöffnung des Maskenanwenders in Verbindung stehendem Maskeninnenraum, wobei die Abdeckeinrichtung als luftdurchlässige Struktur ausgebildet ist und aus einem flexiblen Material gefertigt ist, das unter Wirkung des Druckes im Maskeninnenraum aufgespannt ist.

Dadurch wird es auf vorteilhafte Weise möglich, eine Atemmaskenanordnung zu schaffen, die eine Gasabströmfläche bereitstellt welche eine diffuse Gasabströmung bei geringer Geräuschemission ermöglicht.

Vorzugsweise ist die Abdeckeinrichtung aus einem luftdurchlässigen Gewebematerial, insbesondere Gore-Tex -Material gefertigt. Alternativ hierzu, oder auch in Kombination mit dieser Maßnahme ist es auch möglich, die Abdeckeinrichtung aus einem Porösmaterial zu fertigen.

Die Luftdurchlässigkeit des luftdurchlässigen Materials und die Fläche des hierdurch definierten Abschnitts ist vorzugsweise so gewählt, dass ein hinreichender Gasabstrom aus dem Maskeninneraum gewährleistet ist.

Die Abdeckeinrichtung oder die Dichtlippeneinrichtung kann mit einer Kopfbandeinrichtung gekoppelt sein. Die Kopfbandeinrichtung selbst kann ebenfalls zur Bereitstellung einer Gasabströmfläche herangezogen werden. Diese Gasabströmfläche kann durch eine schlauchartige Zone aus einem luftdurchlässigen Schlauchmaterial gebildet sein.

Die Dichtlippeneinrichtung ist vorzugsweise an die Abdeckeinrichtung angeklebt oder anvulkanisiert oder angespritzt. Es ist auch möglich, die Abdeckeinrichtung lösbar mit der Dichtlippeneinrichtung zu koppeln, oder die Dichtlippeneinrichtung integral mit der Abdeckeinrichtung auszubilden. Es ist möglich, die Abdeckeinrichtung durch Stützstrukturen aufzuspannen.

Die Erfindung betrifft auch eine Atemmaske mit einer Kopfbandeinrichtung nach Anspruch 13, wobei die Kopfbandeinrichtung zumindest abschnittsweise aus einem luftdurchlässigen Material gefertigt ist und eine Leitungseinrichtung umfasst, die mit einem durch die Atemmaske definierten Maskeninnenraum in Verbindung steht, derart, dass ein Abstrom von unter Druck stehendem Atemgas aus dem Maskeninnenraum durch jenen kopfbandseitig vorgesehenen luftdurchlässigen Materialabschnitt erfolgen kann.

Die eingangs angegebene Aufgabe kann auch gemäß einem weiteren Beispiel gelöst werden, durch eine Atemmaskenanordnung mit einem Gewölbekörper, einer Dichtlippeneinrichtung zur Auflage auf der Gesichtsfläche eines Maskenanwenders, einer Atemgasleitungseinrichtung zur Zuleitung von Atemgas zu einem von dem Gewölbekörper begrenzten und mit der Nasen- und/oder Mundöffnung des Maskenanwenders in Verbindung stehendem Maskeninnenraum, wobei im Zusammenspiel mit dem Gewölbekörper ein Luftführungspfad definiert ist, der sich von einem Atemgaseintrittsbereich zu einem Atemgasaustrittsbereich erstreckt, und sich zumindest abschnittsweise entlang der den Gewölbekörper begrenzenden Wandung erstreckt. Dieses Beispiel gehört aber nich zu der Erfindung.

Dadurch wird es auf vorteilhafte Weise möglich, eine günstig zu reinigende Atemmaskenanordnung zu schaffen, die sich durch eine geringe Geräuschemission und ein geringes Totraumvolumen auszeichnet.

### Kurzbeschreibung der Zeichnungen

Weitere Einzelheiten und Merkmale der Erfindung ergeben sich aus der nachfolgenden Beschreibung in Verbindung mit der Zeichnung. Es zeigt:
- **Fig. 1**: eine Skizze zur Erläuterung einer ersten Ausführungsform einer erfindungsgemäßen Atemmaske;
- **Fig. 2**: eine Skizze zur Erläuterung einer zweiten Ausführungsform einer Atemmaske;
- **Fig. 3**: eine Skizze zur Erläuterung eines Gewebematerialabschnitts;
- **Fig. 4**: eine Skizze zur Erläuterung der durch lockere Kette/Schuss-Bindung bei einem Gewebematerial erreichten Mikroporenstruktur.
- **Fig. 5**: eine Skizze zur Erläuterung eines aus einem Gewebematerial gebildeten Auslasseinsatzes für eine Maskenhartschale.
- **Fig. 6**: eine Skizze zur Erläuterung einer Ausführungsform der Erfindung gemäß dem zweiten Lösungsansatz;
- **Fig. 7**: eine Skizze zur Erläuterung eines bevorzugten Aufbaues einer Hartschale und eines zum Einsatz hierin vorgesehenen Einsatzelementes;
- **Fig. 8a**: eine Skizze zur Erläuterung einer bevorzugten Ausgestaltung eines Einsatzelementes der Applikationsstruktur der vorangehend gezeigten Atemmaskenanordnung;
- **Fig. 8b**: eine Skizze zur Erläuterung einer bevorzugten Innengestaltung einer Hartschale mit einem Aufnahmeabschnitt zur Aufnahme des Einsatzelementes nach Figur 8a.

### Ausführliche Beschreibung der Zeichnungen

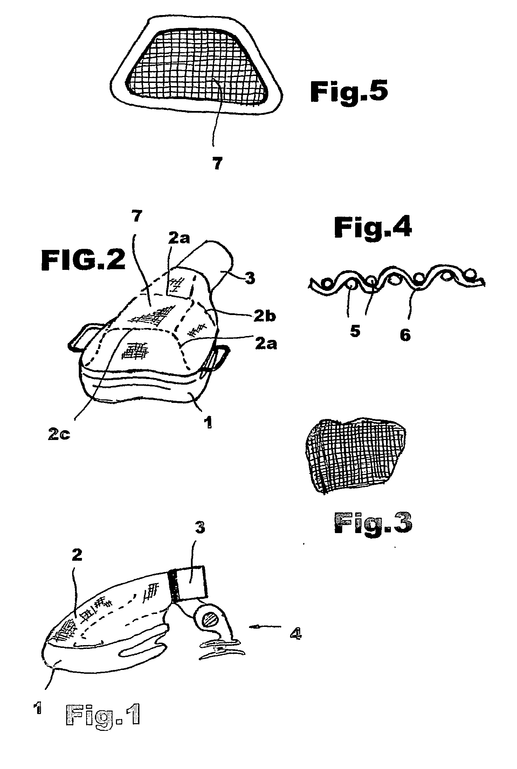

Die in Fig. 1 dargestellte Atemmaskenanordnung umfasst eine, aus einem Elastomermaterial, insbesondere Silikonkautschuk gefertigte Dichtlippeneinrichtung 1 und eine Abdeckeinrichtung 2. Die Dichtlippeneinrichtung 1 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 1 hat hierbei eine im wesentlichen sattelförmige Silhouette.

Die Abdeckeinrichtung 2 ist derart ausgebildet, dass diese im Zusammenspiel mit der Dichtlippeneinrichtung 1 einen Maskeninnenraum definiert. Der Maskeninnenraum steht mit einer Atemgasleitungseinrichtung 3 zur Zuleitung von Atemgas zu jenem von der Abdeckeinrichtung definiertem und mit der Nasen- und/oder Mundöffnung des Maskenanwenders in Verbindung. Die Abdeckeinrichtung 2 ist als luftdurchlässige Gewebestruktur ausgebildet. Die Atemgasleitungseinrichtung 3 bildet einen Anschlussstutzen zur Ankoppelung eines Atemgasschlauches. Die gezeigte Atemmaskenanordnung dient der Zufuhr von Atemgas auf einem Druckpegel der über dem Umgebungsdruck liegt. Die Abdeckeinrichtung 2 wird unter Wirkung des Druckes im Maskeninnenraum zwischen ihren randseitigen Anbindungsstellen, d.h. der Dichtlippeneinrichtung 2 und der Atemgasleitungseinrichtung 3 aufgespannt.

Figur 2 zeigt eine weitere Variante einer Atemmaske. Bei dieser Maske ist die Abdeckeinrichtung 2 durch Nahtabschnitte 2a, 2b, 2c, 2d abgesteppt ausgebildet. Der Verlauf der Nahtabschnitte, sowie die Gestalt der zwischen diesen Nahtabschnitten liegenden Gewebezonen, ist derart abgestimmt, dass die Abdeckeinrichtung unter Wirkung des Atemgasdruckes eine definierte Gestalt erreicht.

Es ist auch möglich, die Abdeckeinrichtung auf eine Rippenstruktur aufzusetzen, oder als Einsatzelement für eine Hartschale auszubilden.

Anstelle eines Gewebemateriales können auch Vlies- oder Filtermaterialien oder anderweitige Porösmaterialien, z.B. mikroperforierte Kunststofffolien Anwendung finden.

Figur 3 zeigt einen Ausschnitt einer aus einem Gewebematerial gefertigten Abdeckeinrichtung.

Figur 4 zeigt Kettfäden 5 und Schussfäden 6 des Gewebeabschnitts nach Figur 3. Zwischen den Kett- und Schussfäden sind Zwischenräume definiert durch welche CO2 befrachtetes Atemgas aus dem Maskeninnenraum entweichen kann.

Figur 5 zeigt ein Einsatzelement für eine Maskenhartschale. Das Einsatzelement umfasst eine Poröszone 7 die aus einem luftdurchlässigen Material gefertigt ist. Über diese Poröszone kann CO2 befrachtetes Atemgas aus dem Maskeninnenraum entweichen.

Die Befestigung der beschriebenen Atemmasken kann durch Kopfbandeinrichtungen erfolgen. Diese Kopfbandeinrichtungen können zur Ableitung von Atemgas herangezogen werden indem diese luftdurchlässige Zonen aufweisen, die über eine Leitungseinrichtung mit dem Maskeninnenraum in Verbindung stehen.

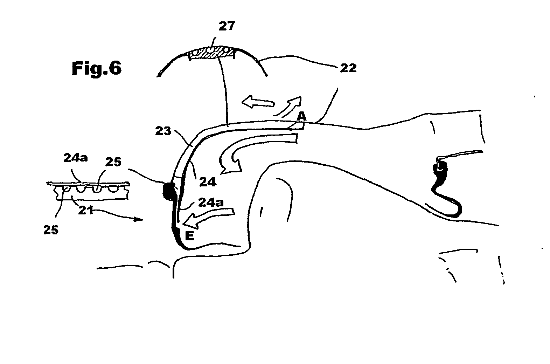

Die in Fig. 6 dargestellte Atemmaskenanordnung umfasst eine, aus einem Elastomermaterial, insbesondere Silikonkautschuk gefertigte Dichtlippeneinrichtung 21 und einen Gewölbekörper 22. Die Dichtlippeneinrichtung 21 ist derart ausgebildet, dass diese eine, zur Aufnahme des Nasenbereichs eines Maskenanwenders vorgesehene Aufnahmeöffnung umsäumt und hierbei vorzugsweise den Nasenrücken sowie den Oberlippenbereich des Maskenanwenders überquert. Die Dichtlippeneinrichtung 21 hat hierbei eine im wesentlichen sattelförmige Silhouette.

Der Gewölbekörpers ist derart ausgebildet, dass dieser einen Luftführungspfad 23 definiert, der sich von einem Atemgaseintrittsbereich E zu einem Atemgasaustrittsbereich A erstreckt, und sich zumindest abschnittsweise entlang einer den Gewölbekörper 22 begrenzenden Wandung erstreckt.

Der Luftführungspfad 23 ist zum Maskeninnenbereich hin durch ein Einsatzelement 24 begrenzt. Weiterhin ist der Gewölbekörper 22 mit einer Fixiereinrichtung versehen, zur Anbringung des Einsatzelementes 24. An dem Einsatzelement 24 ist ein Abdeckabschnitt 24a ausgebildet, der auf einer Kanalrillenstruktur 25 aufliegt, die im Innenbereich der Dichtlippeneinrichtung 21 ausgebildet ist.

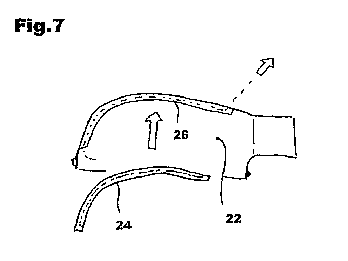

Wie aus Figur 7 ersichtlich, ist das Einsatzelement 24 im Innenbereich des Gewölbekörpers 22 anbringbar ist. Im Innenbereich des Gewölbekörpers 22 ist ein Aufnahmeabschnitt 26 ausgebildet ist, zur Aufnahme des Einsatzelementes 24.

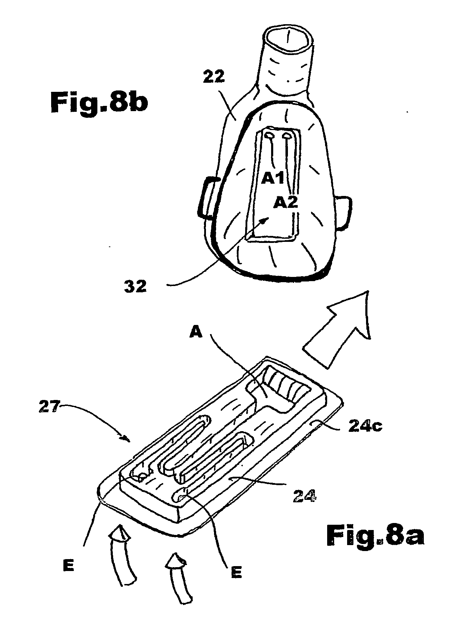

Wie aus Figur 8a ersichtlich sind in dem Einsatzelement 24 Kanalstrukturen 27 ausgebildet. Die Kanalstrukturen 27 sind derart abgestimmt, dass sich ein definierter Strömungswiderstand ergibt. Die Kanalstrukturen 27 erstrecken sich von einem Eintrittsbereich E zu einem Austrittsbereich A. Die Kanalabschnitte 27 sind im montierten Zustand von der Wandung des Gewölbekörpers 22 abgedeckt. Das Einsatzelement 24 ist aus einem Elastomermaterial gefertigt. In dem Einsatzelement 24 sind durch die Kanalabschnitte 27 Labyrinthstrukturen ausgebildet. Das Einsatzelement 24 ist durch Klemmung mit dem Gewölbekörper koppelbar. Zur zusätzlichen Abdichtung ist eine umlaufende Dichtlippe 24c an dem Einsatzelement 24 ausgebildet. Das hier gezeigte Einsatzelement ist in den in Figur 8b gezeigten Gewölbekörper 22 von innen einsetzbar.

Wie aus Figur 8b ersichtlich befinden sich in dem Gewölbekörper 22 Austrittsöffnungen A1, A2, die mit dem Austrittsbereich A des Einsatzelementes übereinkommen und eine Abströmung der verbrauchten Atemluft ermöglichen. Der Gewölbekörper 22 selbst ist bei diesem Ausführungsbeispiel aus einem thermoplastischen Kunststoffmaterial gefertigt. Der zur Aufnahme des Einsatzelementes 24 vorgesehene Aufnahmebereich 32 kann durch eine Vertiefung oder durch eine Umfangswandung definiert sein.

Es ist auch möglich, die Fixierung des Einsatzelementes durch Zapfen oder anderweitige Haltemittel zu bewerkstelligen.

Das Einsatzelement ist hier durch einen flach rechteckförmigen Grundkörper gebildet. Es ist auch möglich, das Einsatzelement anderweitig, insbesondere zylinderscheibenartig oder als Polygon-Prisma zu gestalten. Es ist auch möglich, das Einsatzelement so auszugestalten, dass sich in Abhängigkeit von seiner Position an dem Gewölbekörper bestimmte Drosselwirkungen ergeben.

Das Einsatzelement kann auch so gestaltet sein, dass sich in Abhängigkeit vom Innendruck unterschiedliche Strömungswiderstände, insbesondere durch Verformung der Kanalabschnitte 27 ergeben.

## Patentansprüche

1. Atemmaskenanordnung mit einer Dichtlippeneinrichtung (1) zur Auflage auf der Gesichtsfläche eines Maskenanwenders, einer Abdeckeinrichtung (2) die im Zusammenspiel mit der Dichtlippeneinrichtung (1) einen Maskeninnenraum definiert, einer Atemgasleitungseinrichtung (3) zur Zuleitung von Atemgas zu jenem von der Abdeckeinrichtung (2) definierten und mit der Nasen- und/oder Mundöffnung des Maskenanwenders in Verbindung stehenden Maskeninnenraum, **dadurch gekennzeichnet, dass** die Abdeckeinrichtung (2) als luftdurchlässige Struktur ausgebildet ist, und dass die Abdeckeinrichtung (2) aus einem flexiblen Material gefertigt ist, das unter Wirkung des Druckes im Maskeninnenraum aufgespannt ist.

2. Atemmaskenanordnung nach Anspruch 1, **dadurch gekennzeichnet, dass** die Abdeckeinrichtung (2) aus einem luftdurchlässigen Gewebematerial gefertigt ist.

3. Atemmaskenanordnung nach Anspruch 1, **dadurch gekennzeichnet, dass** die Abdeckeinrichtung aus einem Porösmaterial gefertigt ist.

4. Atemmaskenanordnung nach wenigstens einem der Ansprüche 1 bis 3, **dadurch gekennzeichnet, dass** die Abdeckeinrichtung (2) aus einem Gore-Tex-Material gefertigt ist.

5. Atemmaskenanordnung nach wenigstens einem der Ansprüche 1 bis 4, **dadurch gekennzeichnet, dass** die Luftdurchlässigkeit des luftdurchlässigen Materials und die Fläche des hierdurch definierten Abschnitts so gewählt ist, dass ein hinreichender Gasabstrom aus dem Maskeninnenraum gewährleistet ist.

6. Atemmaskenanordnung nach wenigstens einem der Ansprüche 1 bis 5, **dadurch gekennzeichnet, dass** die Abdeckeinrichtung (2) mit einer Kopfbandeinrichtung gekoppelt ist.

7. Atemmaskenanordnung nach Anspruch 6, **dadurch gekennzeichnet, dass** die Kopfbandeinrichtung ebenfalls zur Bereitstellung einer Gasabströmfläche herangezogen wird.

8. Atemmaskenanordnung nach wenigstens einem der Ansprüche 1 bis 7, **dadurch gekennzeichnet, dass** die Dichtlippeneinrichtung (1) an die Abdeckeinrichtung (2) angeklebt oder anvulkanisiert oder angespritzt ist.

9. Atemmaskenanordnung nach wenigstens einem der Ansprüche 1 bis 7, **dadurch gekennzeichnet, dass** die Abdeckeinrichtung (2) lösbar mit der Dichtlippeneinrichtung (1) gekoppelt ist.

10. Atemmaskenanordnung nach wenigstens einem der Ansprüche 1 bis 7, **dadurch gekennzeichnet, dass** die Dichtlippeneinrichtung (1) integral mit der Abdeckeinrichtung (2) ausgebildet ist.

11. Atemmaskenanordnung nach wenigstens einem der Ansprüche 1 bis 10, **dadurch gekennzeichnet, dass** Stützstrukturen vorgesehen sind, zum Aufspannen der Abdeckeinrichtung (2).

12. Atemmaskenanordnung nach Anspruch 11, **dadurch gekennzeichnet, dass** die Stützstrukturen Teil einer Skelettstruktur bilden.

13. Atemmaskenanordnung nach wenigstens einem der Ansprüche 1-12 mit einer Kopfbandeinrichtung, wobei die Kopfbandeinrichtung, zumindest abschnittsweise aus einem luftdurchlässigen Material gefertigt ist und eine Leitungseinrichtung umfasst, die mit einem durch die Atemmaske definierten Maskeninnenraum in Verbindung steht, derart, dass ein Abstrom von unter Druck stehendem Atemgas aus dem Maskeninnenraum durch jenen kopfbandseitig vorgesehenen luftdurchlässigen Materialabschnitt erfolgen kann.

## Claims

1. A respiratory mask arrangement comprising a sealing lip device (1) for resting on a facial surface of a mask user, a covering device (2) which in cooperation with the sealing lip device (1) defines a mask interior, a respiratory gas conduit (3) for delivering respiratory gas to the mask interior defined by the covering device (2) and communicating with the nostril and/or oral opening of the mask user, **characterized in that** the covering device (2) is embodied as an air-permeable structure and that the covering device (2) is made from a flexible material which is deployed in the mask interior under the influence of the pressure.

2. The respiratory mask arrangement according to claim 1, **characterized in that** the covering device (2) is made from an air-permeable woven material.

3. The respiratory mask arrangement, according to claim 1, **characterized in that** the covering device is made from a porous material.

4. The respiratory mask arrangement according to at least one of claims 1 to 3, **characterized in that** the covering device (2) is made from a Gore Tex material.

5. The respiratory mask arrangement according to at least one of claims 1 to 4, **characterized in that** the air permeability of the air-permeable material and the area of the portion defined thereby are selected such that a sufficient outflow of gas from the mask interior is assured.

6. The respiratory mask arrangement according to at least one of claims 1 to 5, **characterized in that** the covering device (2) is coupled to a headband device.

7. The respiratory mask arrangement according to claim 6, **characterized in that** the headband device is also used for providing a gas outflow area.

8. The respiratory mask arrangement according to at least one of claims 1 to 7, **characterized in that** the sealing lip device (1) is glued or vulcanized or sprayed onto the covering device (2).

9. The respiratory mask arrangement according to at least one of claims 1 to 7, **characterized in that** the covering device (2) is detachably coupled with the sealing lip device (1).

10. The respiratory mask arrangement according to at least one of claims 1 to 7, **characterized in that** the sealing lip device (1) is embodied integrally with the covering device (2).

11. The respiratory mask arrangement according to at least one of claims 1 to 10, **characterized in that** supporting structures are provided for deploying the covering device (2).

12. The respiratory mask arrangement according to claim 11, **characterized in that** the supporting structures form part of a skeleton structure.

13. The respiratory mask arrangement according to at least one of claims 1 to 12 comprising a headband device, wherein the headband device is made at least in portions from an air-permeable material and comprises a conduit device which communicates with a mask interior defined by the respiratory mask so that pressurized respiratory gas can flow out of the mask interior through said air-permeable material portion provided at the headband.

## Revendications

1. Unité de masque respiratoire avec un dispositif de lèvre d'étanchéité (1) à appliquer contre la surface du visage d'un utilisateur de masque, un dispositif de recouvrement (2) qui définit un espace intérieur du masque en coopération avec le dispositif de lèvre d'étanchéité (1), un dispositif de conduite de gaz respiratoire (3) pour l'amenée de gaz respiratoire vers ledit espace intérieur du masque défini par le dispositif de recouvrement (2) et en liaison avec l'ouverture nasale et/ou buccale de l'utilisateur de masque, **caractérisée en ce que** le dispositif de recouvrement (2) est réalisé comme structure perméable à l'air, et **en ce que** le dispositif de recouvrement (2) est fabriqué dans un matériau souple tendu sous l'effet de la pression dans l'espace intérieur du masque.

2. Unité de masque respiratoire selon la revendication 1, **caractérisée en ce que** le dispositif de recouvrement (2) est fabriqué dans un matériau tissé perméable à l'air.

3. Unité de masque respiratoire selon la revendication 1, **caractérisée en ce que** le dispositif de recouvrement est fabriqué dans un matériau poreux.

4. Unité de masque respiratoire selon au moins une des revendications 1 à 3, **caractérisée en ce que** le dispositif de recouvrement (2) est fabriqué dans un matériau Gore-Tex.

5. Unité de masque respiratoire selon au moins une des revendications 1 à 4, **caractérisée en ce que** la perméabilité à l'air du matériau perméable à l'air et la surface de la partie ainsi définie sont sélectionnées de manière à garantir un échappement gazeux suffisant depuis l'espace intérieur du masque.

6. Unité de masque respiratoire selon au moins une des revendications 1 à 5, **caractérisée en ce que** le dispositif de recouvrement (2) est accouplé à un dispositif serre-tête.

7. Unité de masque respiratoire selon la revendication 6, **caractérisée en ce que** le dispositif serre-tête est également utilisé pour obtenir une surface d'échappement gazeux.

8. Unité de masque respiratoire selon au moins une des revendications 1 à 7, **caractérisée en ce que** le dispositif de lèvre d'étanchéité (1) est collé, ou vulcanisé, ou moulé par injection contre le dispositif de recouvrement (2).

9. Unité de masque respiratoire selon au moins une des revendications 1 à 7, **caractérisée en ce que** le dispositif de recouvrement (2) est accouplé de manière amovible au,dispositif de lèvre d'étanchéité (1).

10. Unité de masque respiratoire selon au moins une des revendications 1 à 7, **caractérisée en ce que** le dispositif de lèvre d'étanchéité (1) est réalisé d'un seul tenant avec le dispositif de recouvrement (2).

11. Unité de masque respiratoire selon au moins une des revendications 1 à 10, **caractérisée en ce que** des structures d'appui sont prévues pour tendre le dispositif de recouvrement (2).

12. Unité de masque respiratoire selon la revendication 11, **caractérisée en ce que** les structures d'appui font partie d'une structure de squelette.

13. Unité de masque respiratoire selon au moins une des revendications 1 à 12 avec un dispositif serre-tête, où ledit dispositif serre-tête est fabriqué au moins partiellement dans un matériau perméable à l'air et comprend un dispositif de conduite en liaison avec un espace intérieur défini par le masque respiratoire, de manière à obtenir un échappement gazeux de gaz respiratoire sous pression depuis l'espace intérieur du masque par ladite partie de matériau perméable à l'air prévue côté serre-tête.
